# EUROPEAN PATENT APPLICATION

(11) **EP 3 791 905 A1**
(43) Date of publication of application: **17.03.2021**
(21) Application number: 19799536.8
(22) Date of filing: 12.03.2019
(51) Int. Cl.: A61M 3/02, A61M 31/00

(54) **DEVICE AND METHOD FOR INJECTING LIQUID DRUG INTO NASAL CAVITY AND PARANASAL SINUSES**

(30) Priority: 11.05.2018 KR 20180054471
(71) Applicant: Fluchem Ltd, Daejeon 34078 (KR)
(72) Inventor: KIM, Dae Whang, Daejeon 34083 (KR); KIM, Seung Shin, Seoul 05658 (KR); PARK, Yang Ok, Daejeon 34083 (KR); KIM, Jung Wook, Daejeon 34082 (KR)
(74) Representative: Gerauer, Marc Philippé
(86) International application number: PCT/KR2019/002848
(87) International publication number: WO 2019/216535

(57) **Abstract**

The present invention relates to a liquid drug injection device for injecting a liquid drug into a nasal cavity and paranasal sinuses through a nostril and, more specifically, to a device for injecting a liquid drug into a nasal cavity and paranasal sinuses, the device being characterized in that: an adaptor, which is formed of an elastic material such that compression and restoration can be carried out and which has an accommodation space therein, is mounted at a discharge part of a liquid drug storage container; a liquid drug stored in the liquid drug storage container is injected into the nasal cavity through the adaptor so as to fill the nasal cavity with the liquid drug; while the nasal cavity is filled with the liquid drug, the liquid drug storage container is repeatedly pressed toward the adaptor and released so as to change the volume of the adaptor; the change in the volume of the adaptor generates turbulence or sloshing of the liquid drug filled in the nasal cavity; and the generated turbulence and sloshing promotes the liquid drug to contact the surface of the nasal cavity and flow toward the paranasal sinuses so as to enhance the effect of cleaning the surfaces of the nasal cavity and the paranasal sinuses and injecting the liquid drug, and to increase the effectiveness of preventing and treating nasal diseases.

## Description

### Technical Field

The present invention relates to a device and method for injection of a medication into the nasal cavity and paranasal sinuses through a nostril and, more specifically, to a device and method for injection of a medication into the nasal cavity and paranasal sinuses, wherein: an adaptor formed of an elastic material to allow compression and restoration and having an accommodation space therein is mounted at an inlet of a medication storage container; a medication stored in the medication storage container is injected into the nasal cavity through the adaptor; while the nasal cavity is filled with the medication, the medication storage container is repeatedly pressed toward the adaptor and released so as to change the volume of the adaptor; the change in the volume of the adaptor generates turbulence or sloshing of the medication in the nasal cavity; and the generated turbulence and sloshing promotes the medication to contact the surface of the nasal cavity and flow toward the paranasal sinuses so as to enhance the effect of cleaning the surfaces of the nasal cavity and the paranasal sinuses and injecting the medication and to increase the effectiveness of preventing and treating nasal diseases.

### Background Art

Breathing brings about a lot of microbes into contact with the nasal cavity every day, which may cause the onset of various diseases in the nasal cavity. Representative of them is sinusitis, which affect 10%-30% of the population in the United States and the Europe annually. Women are more vulnerable to the disease than men. In the United States, the onset of sinusitis occurs in about 12.5% of the population every year with annual medical expenses therefor amounting to US $11 billion.

Sinusitis, which is inflammation in the paranasal sinuses, is also known as rhinosinusitis because it always occurs together with the nasal inflammation known as rhinitis. Rhinosinusitis is an inflammatory disorder in the mucous membranes that line the nasal cavity and paranasal sinuses. Rhinosinusitis may be acute or chronic and can be caused by virus, bacteria, fungi, allergies, or autoimmune reactions. As much as 90% of the cases of adult rhinitis are accounted for by viral infection, with bacteria accounting for the remaining 10% of the adult patients. In particular, when infected with the common cold virus or influenza virus, a person is likely to suffer acute rhinosinusitis and, would be greatly prohibited from living a normal life. In case where the onset of rhinosinusitis is induced by common cold or influenza viruses, the viruses should adhere to the surfaces of host cells in order to penetrate the cells. The cells having receptors to which the viruses can easily adhere are distributed particularly on the surfaces of the nasal cavity and the paranasal sinuses, and account for primary infection sites at which the viruses adhere and replicate.

In order to remedy rhinosinusitis, the injection of a medication into the nasal cavity and the paranasal sinuses is needed. Many devices have been developed and are now marketed. However, devices or methods that can effectively treat rhinosinusitis are not yet known. Irrigation of the nasal cavity is most commonly used and is exemplified by a flood irrigation in which a medication is superfluously poured into the nasal cavity to clean the nasal cavity.

Flood irrigation differs significantly from the practice of inhaling an atomized mist into the nose. Rinsing with flood irrigation is performed by injecting the medication into one nostril and concurrently expelling the excess medication from the other nostril. During flood irrigation, the vast majority (≥95%) of fluid taken in is expelled immediately after the contaminants have been rinsed out. Alternately, flood irrigation is performed by ingesting the medication into both nostrils simultaneously and having the excess flow to the mouth. Flood irrigation is to physically rinse the mucous membrane in the nasal cavity.

Nowadays, there are various devices and techniques to facilitate the rinsing by flood irrigation of the nasal cavity. The liquid delivery devices may be generally divided into two major commercial categories: 1) simple devices which dispense a continuous low-pressure stream of fluid from a squeeze bottle, deformable bulb, bellows container, shower head connection, gravity feed, etc., and b) devices which use a motorized pump or other complex and expensive electromechanical apparatus to provide a pulsating stream of fluid.

Typically, the devices which dispense a continuous low-pressure stream of irrigant typically are relatively low in cost, but unfortunately, they offer a less than optimal cleaning ability due to the tendency of the continuous stream to form laminar flow paths across the surfaces to be rinsed and due to the surfaces not being deformed and agitated by the smooth flow stream. These continuous stream devices are also ineffectual in injecting liquid medications or irrigants into paranasal sinus cavities.

In order to develop devices for injecting a medication into paranasal sinuses, which solve such problems, a lot of attempts have been made over the last several decades to so, including application of vibration, sonic waves, or pressures to the mist by use of devices provided with an electronic element. Nevertheless, none of the attempts have successfully developed effective devices for injecting a medication into paranasal sinuses.

This is attributed mainly to the anatomical structure of the paranasal sinuses. The paranasal sinuses, which lie within the facial bones, are joined to the nasal cavity via small orifices, called ostia, but amount to closed spaces where ventilation is almost impossible.

Previous studies reported that pressure modulation promotes the entry of a medication into the non-ventilated space of particles. However, it was also reported that in consideration of the narrow, long, and maze-like structure of the ostiomeatal complex in paranasal sinuses, the application of constant mechanical vibration or a certain frequency of sonic waves is not effective for injecting a medication into the paranasal sinuses. Furthermore, when the nose is completely congested due to chronic sinusitis or acute rhinitis, it is impossible to inject a medication using such methods. Up to date, very little is known about effective methods for local administration of a medication into paranasal sinuses.

This is because the entrance to the paranasal sinus is as small as 1-5 mm in diameter. Without vibrations or with a nasal pump sprayer, the delivery is conducted at an efficiency of 0.2% only. Moller et al. reported in PLoS One 8, e74991(2013) that aerosol pulsation at a frequency of 25 Hz resulted in sinus deposition at a rate of about 4% for sinusitis patients and at a rate of about 7% for healthy volunteers. In Int. J. Pharm. 494, 227 (2015), Leclerc et al. examined the influence of acoustic waves on drug delivery to the maxillary sinus in a plastinated human cast. As a result, it was reported that the efficiency of delivery varies as a function of frequency of the vibrating acoustic airflow and the geometry of the ostia in the maxillary sinus. That is, the mode of vibrating acoustic airflow must varies depending on sizes and structures of the ostia. However, it is practically impossible to apply different frequency modes depending on patients or disease conditions.

In addition, the naval structure differs from one person to another and there is a structural difference for the opposite spaces even in one nose. Also, differences in nasal structure are caused by nasal septal deviation, turbinate hypertrophy, and positions and sizes of the nasal valve. Given, rhinitis, polyposis, periodical nasal obstruction, and the like do not allow the administration of medications to desired sites, which results in undesirable outcomes. Moreover, the onset of common cold or influenza infection causes serious nasal obstruction, making it impossible to administer medications through a nostril. If unable to receive a medication, the affected nasal site remains in an infected state, with the continuous supply of an infectious source thereto.

In the cranium, there are paranasal sinuses communicating the nasal cavity and lined with mucous membranes, such as maxillary sinus, frontal sinus, ethmoid sinus, sphenoid sinus. The paranasal sinuses function to heat the air upon breathing and make the voice resonate. Upon the onset of common cold, influenza, or the like, the bacteria or virus also infects the paranasal sinuses. However, medication administration by conventional practices is difficult for the paranasal sinuses as well as the folded internal surface of the turbinate positioned in the nasal cavity. The rinsing mode of flowing a medication into one nasal cavity has a difficulty in drug delivery into the paranasal sinuses outwardly positioned and in rinsing even the inner side of the turbinate. Thus, the rinsing mode may reduce the symptom temporarily, but cannot cure the disorder because infection spreads from an unrinsed site.

Therefore, there is a need for a device and method of novel concept that can inject a medication into the paranasal sinuses. In order to inject a medication into paranasal sinuses, the device of novel concept should meet the following requirements: the device provides a low to a high pressure so that it can be generally available for all cases irrespective of patients' states or anatomical differences; the pressure is changed erratically to introduce a medication into the paranasal sinuses through the ostiomeatal complex; the device is able to form turbulence to generate fluid oscillation and pressure modulation in complex; the fluid being injected into the nasal cavity is fast and strong enough to move though the hypertrophic turbinate; after use, the device is easy to cleanse and disinfect and can be prevented from secondary infection upon reuse; and the device can be prevented from being contaminated and is small, convenient for use, and inexpensive; and the device allows a small amount of a medication to be applied to the treatment of all kinds of rhinosinusitis. These requirements need a device and method of new concept.

Korean Utility Number 20-0482070 (issued December 06, 2016; hereinafter referred to "prior art document 1") suggests a device which has a filter for intranasal injection of a medication. The device is mounted at the front end of a medication injector and has a filter installed in the inner channel thereof such that the medication is filtered before being sprayed. Prior art document 1, although providing the function of injecting a filtered medication, adopts a spraying mode and thus is still a difficulty in rinsing the internal turbinate and the paranasal sinuses.

Korean Patent Number 10-0977070 (issued on October 13, 2010; hereinafter referred to as "prior art document 2") proposes a spray nozzle for intranasal rinse and an intranasal rinsing apparatus using same. The apparatus of prior art document 2 is structured such that a cleansing solution stored in a container is delivered via a channel path to a discharge member from which the cleansing solution is sprayed, wherein a pressing portion is formed on the channel path, whereby the spraying pressure of the cleansing water is controlled depending on the pressure applied to the pressing portion. Prior art document 2, although able to control the supply intensity of a medication, has a difficulty in rinsing the internal turbinate or paranasal sinuses because it adopts a spraying mode.

Therefore, a need is arising for a novel medication injecting device that is sanitary and simple in structure and can rinse the folded structure of the turbinate and the paranasal sinuses, which are different spaces, even in a nasal obstruction condition.

### Detailed Description of the Invention

### Technical Problem

In order to solve the problems encountered in the conventional arts, the present invention proposes a device and method for injecting a medication into the nasal cavity and paranasal sinuses, which is convenient for use by a patient himself or herself, inexpensive, sanitary, and effective, with which the patient can inject the medication into the inferior nasal cavity, with the his or her head tilted to one lateral side and in which the medication loaded into the nasal cavity is allowed to flow or undergo turbulence, thereby rinsing the narrow regions such as gaps between turbinates, intranasal sinuses and so on.

### Technical Solution

In order to achieve the purpose, the present invention provides a device for injecting a medication into the nasal cavity and the paranasal sinus through a nostril, the device comprising:
a) a medication storage container, having an inlet at one side thereof, for containing the medication therein; and b) an adaptor, comprising: an adaptor body, formed of one elastic material selected from silicone, rubber, and an elastic plastic material, for elastically moving to volumetrically reduce or restore the internal accommodating space thereof depending on external pressures applied thereto; a connector formed at the rear end of the adaptor body and connected to the inlet of the medication storage container; and an outlet, formed at the front end of the adaptor body, for forward discharging the medication provided from the medication storage container, wherein while the nasal cavity is filled with the medication by injection through a nostril, pressure application and release are repeatedly carried out for the adaptor to transfer the volume change of the internal accommodation space according to the pressure change to the medication in the nasal cavity so that the medication undergoes turbulence, vortex, pulsation, or sloshing, whereby the nasal cavity and the paranasal sinuses can be rinsed and administered the medication.

The adaptor may have one of: a structure in which a front side protrudes to come into close contact with a nostril and a rear side of the adaptor has a wide heart form, a hemisphere form, a circular cone form, or a circular truncated cone form; a polygonal structure in which a front side is partially formed into a circular cone or a circular truncated cone; or a polygonal structure in which a front side is partially formed into a circular cone or a circular truncated cone, with the slant face of the circular cone or the circular truncated cone being concave or convex.

In addition, the adaptor may be an integrated structure or an assembled structure that can be detachably mounted horizontally or vertically.

Also, the adaptor body may be formed of an elastic material and may be 10-60 mm in length, 10-50 mm in maximum diameter, 1-50 ml in accommodation space volume, and 0.1-5 mm in thickness; the connector may be 1-5 mm in inner diameter and 0-10 mm in length; and the outlet may be 2-15 mm in diameter.

Furthermore, the connector of the adaptor may be formed by perforating the adaptor body or into an inwardly internalized structure or an outwardly protruding structure and may be engaged with the inlet of the medication storage container by screwing or press fitting.

On the inner surface of the adaptor, a plurality of reinforcement strips, each extending from the outlet to the connector, may be formed at regular distances of space to reinforce a lengthwise elastic restoring force.

The medication storage container may be a syringe or a compression container. The compression container may be formed of one elastic material selected from a silicone, a rubber, and a plastic while the syringe may be formed of a plastic or glass material.

In addition, the medication stored in the medication storage container may be an aqueous 0.6-5% NaCl solution or a solution containing povidone-iodine at a concentration of 0.01-0.3 w/v%.

Moreover, the present invention provides a medication injection method using the medication injection device of the present invention, the method comprising:
a medication injection step in which a medication is injected into a nasal cavity through an adaptor by using a syringe containing the medication and serving as a medication storage container; an adaptor compression and release step in which, while the nasal cavity is filled with the medication, pressures are repeatedly applied to and released from the syringe to discontinuously modulate the volume and pressure in the accommodation space of the adaptor, thereby repeating the backward flow and reentry of the medication loaded to the nasal cavity; and a rinsing step in which turbulence or sloshing occurs in the nasal cavity with the change of the medication stream in speed and pressure upon the backward flow and reentry, thereby rinsing the nasal cavity and paranasal sinuses.

In addition, the method may further comprise a medication injection posture setting step in which the patient's head is tilted by 60-160 degrees to the lateral side, followed by injecting the medication through a lower nostril or in which the patient's head is tilted by 60-160 degrees to the front side and then the patient's face is turned by 60-160 degrees, followed by injecting the medication through a lower nostril, prior to the medication injections step.

In the adaptor compression and release step, the application and release of pressures by the syringe may be carried out at a frequency of 30-300 cycles per minute.

Furthermore, the medication injection method of the present invention may be applied to the treatment or prevention of at least one of rhinitis, sinusitis, common cold, and influenza as well as the generation of thick nasal mucus or nasal obstruction.

### Advantageous Effects

The device and method for injection of a medication into the nasal cavity and paranasal sinuses according the technical solution of the present invention is designed to function as follows:
An adaptor formed of an elastic material is mounted to an inlet of a medication storage container to inject a medication stored in the medication storage container into the nasal cavity therethrough, and while the nasal cavity is filled with the medication, the adaptor is subjected to many cycles of contraction and expansion by iterative pressure application and release.

When the contraction and expansion of the adaptor is iteratively performed, the medication that goes forth and back between the nasal cavity and the adaptor varies in pressure and flow speed, whereby turbulences, vortexes, pulsatile streams or sloshing of the medication loaded to the nasal cavity are generated to rinse every nook and corner on the surfaces of the nasal cavity and paranasal sinuses.

Particularly, the generated turbulences and sloshing promote the access of the medication to narrow gaps between turbinates and to the paranasal sinuses to improve rinsing efficiency, thereby removing infectious pathogens and effectively relieving or treating rhinosinusitis.

### Brief Description of the Drawings

FIG. 1 is a schematic cross-sectional view of a medication injection device according to an embodiment of the present invention.
FIGS. 2a to 2l are cross-sectional views of various forms of an adaptor according to an embodiment of the present invention.
FIGS. 3a and 3b are cross-sectional views of an adaptor according to another embodiment of the present invention.
FIG. 4 is a schematic view of a medication storage container according to another embodiment of the present invention.
FIG. 5 is a schematic view showing the application practice of a medication injection device according to an embodiment of the present invention.

### Best Mode for Carrying Out the Invention

Below, a detailed description will be given of embodiments of the present invention with reference to the accompanying drawings. The present invention may be changed in various forms, and may be implemented as various embodiments. Specific embodiments will be illustrated in the drawings and described in detail in the detailed description. However, it should be understood that the present invention is not limited to the specific embodiments, and the present invention includes all changes, equivalents and substitutions included in the spirit and technical scope of the present invention. In describing the drawings, like reference numerals are used for like elements. In the accompanying drawings, the dimensions of the structures may be enlarged than the actual dimensions for clarity of the invention or may be reduced than the actual dimensions for understanding of the schematic structure.

The terms used in the description are for the purpose of describing particular embodiments only, and are not intended to be limiting of the present invention. The singular forms are intended to include the plural forms as well, unless the context clearly indicates otherwise. It will be further understood that the terms "comprises (comprising)", "includes (including)", or "have (having)" in the present specification specify the presence of features, numerals, steps, operations, elements, components, or combinations thereof stated in the present specification, but do not preclude the presence or addition of one or more other features, numerals, steps, operations, elements, components, or combinations thereof.

If it is not contrarily defined, all terms used herein including technological or scientific terms have the same meanings as those generally understood by a person with ordinary skill in the art. Terms defined in generally used dictionary shall be construed that they have meanings matching those in the context of a related art, and shall not be construed in ideal or excessively formal meanings unless they are clearly defined in the present application.

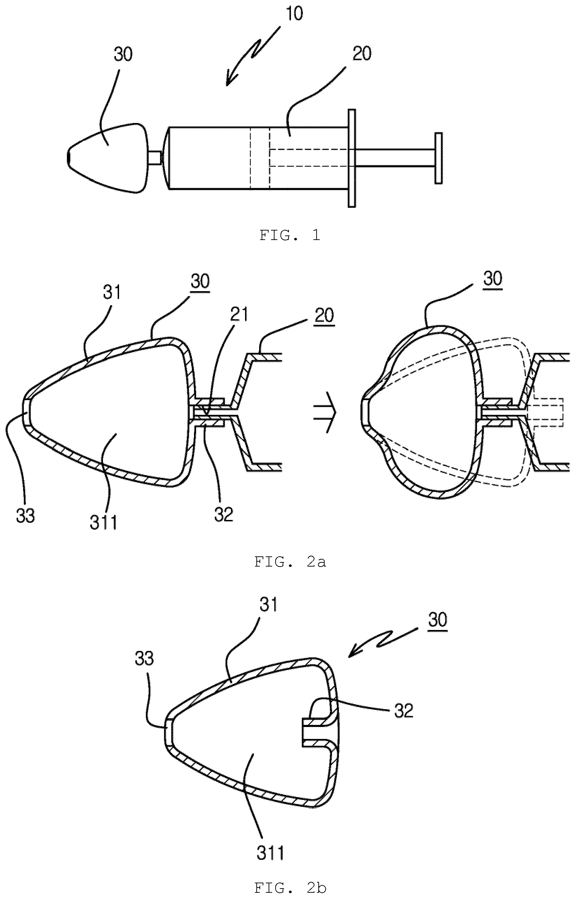

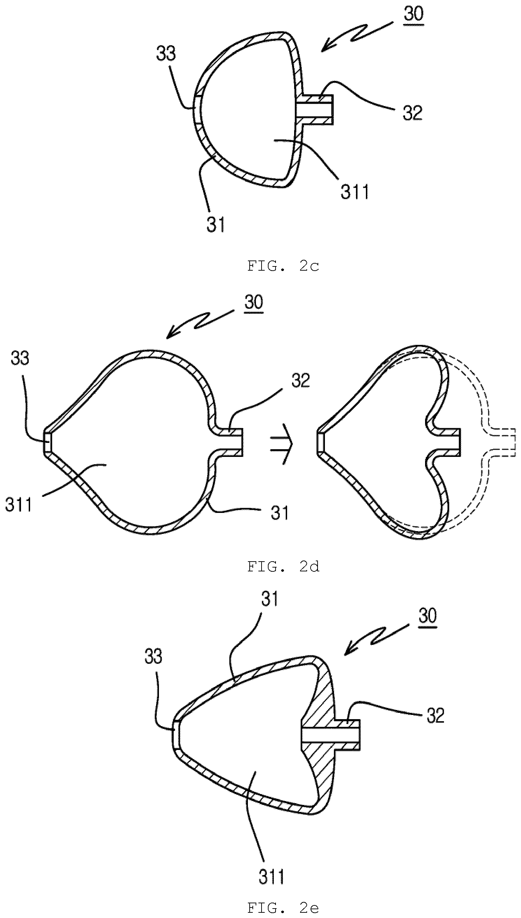

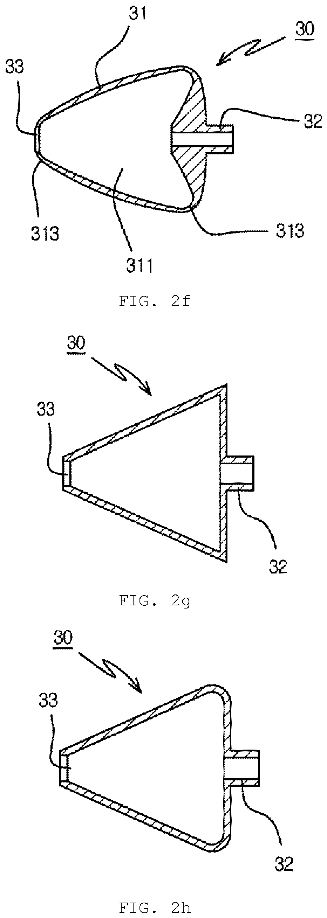

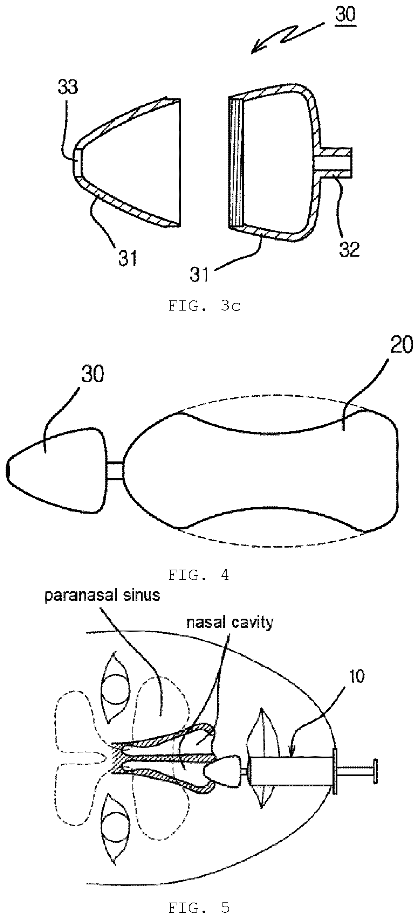

FIG. 1 is a schematic cross-sectional view of a medication injection device according to an embodiment of the present invention, FIGS. 2a to 2l are cross-sectional views of various forms of an adaptor according to an embodiment of the present invention, and FIG. 4 is a schematic view of a medication storage container according to another embodiment of the present invention.

As shown, a medication injection device (10) according to the present invention comprises: a medication storage container (20) for storing a liquid medication therein, which has an inlet (21) formed at a side thereof to discharge the medication therethrough; and an adaptor (30), engaged with the inlet of the medication storage container and made of an elastic material, for introducing the medication discharged from the medication storage container into a nasal cavity through a nostril.

The medication storage container (20) may be formed into a syringe, as can be seen, or into a compression container shown in FIG. 4 to discharge the medication stored therein. In this regard, the medication is preferably stored at one dose in the medication storage container so as to prevent the infection which may occur upon multiple uses. In addition, the compression container may be made of an elastic material such as silicone, rubber, or plastic while the syringe may be made of a plastic or glass material.

In addition, the inlet (21) may be detachably engaged to the adaptor. In this regard, the inlet (21) may have screw threads formed on the outer surface thereof and thus may be screwed to the adaptor. Alternatively, the inlet may have a locking protrusion or groove and thus may be fitted or forcibly fitted to the adaptor.

The adaptor (30) comprises: an adaptor body (31) having an accommodation space (311) formed therein; a connector (32) formed at the rear end of the adaptor body to engage with the inlet of the medication storage container; and an outlet (33) formed at the front end of the adaptor body to forwardly discharge the medication introduced from the rear side.

Being made of an elastic material, the adaptor body (31) can vary in volume so as to reduce and restore the volume of the inner accommodation space upon the application of external forces thereto. In this regard, the external force is applied in the axial direction, which is the forward direction, by the medication storage container connected to the rear end of the adaptor body. The adaptor body is compressed in the axial direction upon application of an external force thereto and restored to the original position upon removal of the external force. Therefore, the adaptor body (31) is provided as a structure in which the rear side is a broad surface to readily receive an external pressure from the medication storage container while the front side has a shape capable of closing various morphologies of nostrils with the lateral face close to the front end when inserted to the nostrils.

Therefore, the adaptor body (31) has a morphological structure in which the rear side is broad and the front side is narrow as shown in FIGS. 2a to 2l, such as a heart shape, a hemisphere shape, a circular cone or circular truncated cone shape, a triangular or polygonal container shape having a circular cone or circular truncated cone formed as a part of the front end thereof, or a triangular or polygonal container shape having a circular cone or circular truncated cone formed as a part of the front end thereof, with the slant face of the circular cone or the circular truncated cone being concave or convex. As shown in FIG. 2d, a compression force transferred from the connector (32) moves the broad surface of the rear site in the horizontal axis direction to rapidly reduce the volume of the inner accommodation space, thereby injecting the medication at a higher pressure.

That is, the morphological structures of the adaptor in FIGS. 2a to 2l are designed to transfer the pressure to the entire lateral wall, thereby expanding the lateral wall outwardly while reducing the volume of the inner accommodation space. However, in the adaptor shown in FIG. 2d, the pressure is not transferred to the lateral wall, but to a part of the rear side. In this regard, the transferred pressure moves the connector portion forward to reduce the volume of the inner accommodation space. Being able to focus the transferred pressure locally, the morphological structure can thus inject the stored medication with the volume reduction of the accommodation space by even a small pressure. At the same pressure, this structure can strongly inject a medication, compared to the other structures because the pressure loss of expanding the lateral wall of the adaptor is prevented.

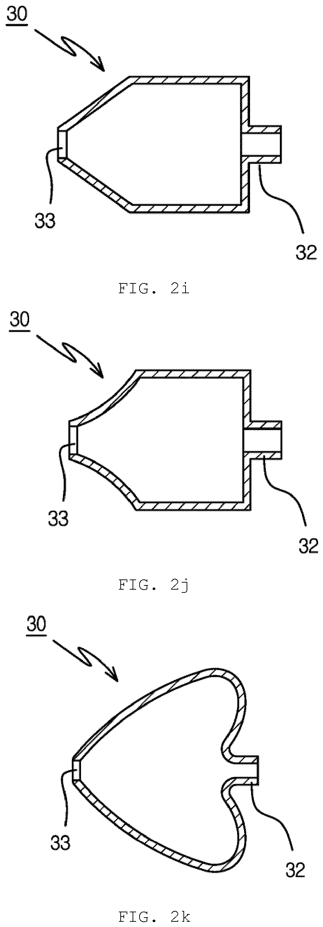

In addition to the aforementioned morphological structure, the adaptor (30) may be formed into triangular or polygonal cones or truncated cones as shown in FIGS. 2g to 2j, and the corner portion at which the rear side having the connector provided therein is connected with the lateral wall may be formed to be angular or round. Furthermore, as shown in FIG. 2i, the adaptor (30) may have a polygonal container structure in which a part of the front side having an outlet is formed into a circular cone or circular truncated cone having a slant face. In addition, as shown in FIG. 2j, the slant face in the structure of FIG. 2i may be concave or convex (not shown) to facilitate the entry of the outer portion to a nostril.

As shown in FIG. 2k, the adaptor may have a heart structure in which the rear side having the connector formed therein is convex and the connecting portion between the connector and the rear side is concave. This heart structure allows the rear side to easily move forth and back when an external force is applied thereto.

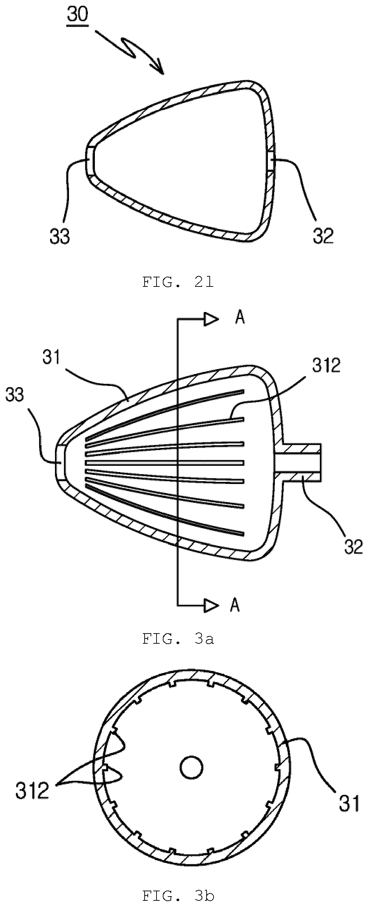

As shown in FIG. 2l, a protruding connector (32) is not provided, but a hole is formed in the adaptor body so that the inlet of the medication storage container may be directly coupled thereto.

In addition, the adaptor according to the present invention may have various morphological structures designed to vary in the accommodation space upon the application of a pressure thereto.

The adaptor body (31) may be formed to have the following dimensions: length 10-60 mm, maximum diameter 10-50 mm, accommodation space volumel-50 ml, and thickness 0.1-5 mm. When the length is shorter than 10 mm, the inner accommodation space is too small in space to produce a discharge pressure sufficient to cause the generation turbulence, vortex, stream, or sloshing of the medication, with the resultant low rinsing effect obtained. When the adaptor body is longer than 60 mm, the excessive length makes it difficult to exert the compression forward, resulting in a loss of the compression force. When the maximum diameter is less than 10 mm, the discharge pressure is lower owing to the reduced volume of the inner accommodation space. In case where the maximum diameter exceeds 50 mm, the outlet is difficult to maintain in the direction toward the inside of the nasal cavity (in the direction parallel to the facial surface) when the adaptor comes into close contact with a nostril, but the outlet faces the inner wall of the nasal cavity so that it is difficult to uniformly transfer a discharge pressure across the nasal cavity. When the accommodation space is less than 1 ml in volume, the accommodation space generates a low discharge pressure and results a low rinsing effect. When the accommodation space exceeds 50 ml in volume, the generation intervals of pulsatile streams in which the discharge pressure periodically varies with repeated compressions are lengthened, which results in lowering a rinsing effect. When the thickness is less than 0.1 mm, the elastic restoring force decreases so that pulsatile streams are difficult to generate with periodic applications of pressures. When the thickness is over 5 mm, the structural strength is high, requiring a great force for compressing the adaptor body. Also, the adaptor applies a great force to the nose, causing a pain when coming to contact with a nostril. Therefore, the dimensions of the adaptor body are preferably established in the ranges.

However, the material thickness of the adaptor body needs not to be consistent across the adaptor body, and may be properly adjusted so as to increase close contact with a nostril or to facilitate the compression and restoration. For example, referring to FIG. 2e, the rear side at which the connector is present may be given an increased thickness so that only the lateral sides of the adaptor body are deformed upon the application of an external force. Referring to FIG. 2f, a deformation part (313), which is thin, may be formed at the front outlet portion and the connection portion between the rear side and the lateral side so as to pre-determine positions at which deformation occurs upon the application of an external force.

The connector (32) is integrated into the adaptor body (31) at the rear end and is structured to engage with the inlet (21) of the medication storage container. The connector (32) may be provided as a pipe body protruding in the rear direction as shown in FIG. 2a, as a pipe body protruding inside the adaptor body as shown in FIG. 2b, or as a hole formed at the rear end of the adaptor body (31) as shown in FIG. 2l. The connector (32) of the adaptor and the inlet (21) of the medication storage container may have screw threads or a locking protrusion or groove on the inner or outer surface thereof so that they can be detachably engaged with each other in a screwing, fitting, or forcibly fitting mode.

In addition, the connector (32) may have an inner diameter of 1-5 mm and a length of 0-10 mm. The inner diameter is large enough to allow the inlet of the medication storage container to internally fit thereto and is not limited to the range. However, when the inner diameter is less than the lower limit, the connector may be folded or damaged. When the inner diameter exceeds the upper limit, a structure in which the connector is formed inside the adaptor body has a relatively small volume of the inner accommodation space, which results in decreasing an amount of effluent medication and a discharge pressure. Thus, the range is preferable.

Within the length range of 0-10 mm, the connector may be provided as various forms capable of engaging with the inlet of the medication storage container. For example, the connector may be a hole to internalize the inlet thereinto or may be designed to internalize the inlet or to be inserted into the inlet.

The outlet (33), formed at the front end of the adaptor body, functions to discharge the medication delivered from the medication storage container (20) therethrough. The front end of the adaptor body at which the outlet is formed is inserted into a nostril while the lateral wall proximal to the front end in the adaptor body comes to close contact with the nostril to close the nostril. Also, the outlet may be formed into a pipe having a predetermined length so as to readily enter the nostril. The end of the outlet may be round to cause no irritations upon contact with the inside of the nostril. To this end, the end of the outer may be provided in a round ring form.

The outlet (33) is preferably formed to have an inner diameter of 2-15 mm. A diameter outside the range allows the medication to be discharged in too large or small an amount to generate turbulence, vortex, or pulsatile streams according to the compression. Thus, the inner diameter is preferably formed within the range.

The adaptor may be made of an elastic material such as silicone, rubber, plastic, or the like. The adaptor may be formed of an elastic material in entirety or in part. In the latter case, only the adaptor body is formed of an elastic material while the connector may made of a hard material.

Referring to FIGS. 3a and 3b, the adaptor (30) may have multiple reinforcement strips (312) formed at regular intervals of distance or angles on the inner surface thereof, each extending from the outlet (33) to the connector (32). The reinforcement strips (312) reinforce the structural strength of the adaptor in the axial direction between the outlet and the connector to increase the elastic restoring force upon the release of the compressive force applied to the adaptor, thereby making a rapid restoration. That is, the reinforcement strips contributes to increasing an elastic restoring force in the axial direction, without increasing the wall thickness of the adaptor.

The adaptor (30) may be an integral form in entirety. Alternatively, as shown in FIG. 3c, the adaptor (30) may be in a separable structure so as to easily wash the inside thereof after use. Given a separable structure, the adopt body (31) may be separated to form a separation face perpendicular to the discharge direction as seen, or separated in the discharge direction of the outlet. Alternatively, the rear portion accounting for the connector portion may be separated to open the inside of the adaptor body, in addition to the separation made at the middle portion of the adaptor. Moreover, the detachable engagement may be achieved by various known methods such as screwing or forcibly fitting. In addition, the adaptor may be separated into multiple fragments as well as two fragments as seen.

The medication injection device (10) of the present invention is designed to inject 0.6 to 5 w/v% brine (aqueous NaCl solution) or a 0.01-0.3 w/v% povidone-iodine solution as a medication into a nasal cavity to effectively prevent or treat an infection, an inflammation, or a disease caused upon the onset of rhinitis, sinusitis, rhinorrhea, nasal obstruction, common cold, seasonal flu, or influenza.

The medication injection device of the present invention, comprising an adaptor made of an elastic material as described above, is used as follows.

After a medication storage container (20) is filled with 10-30 ml of a medication, an adaptor (30) is engaged with an inlet of the medication storage container.

A user's head is tilted in the front direction by 60-160 degrees and preferably 90-120 degrees and then turned toward a lateral side by 60-160 degrees and preferably 90-120 degrees so that the left and right nasal cavities are positioned upper and lower, respectively. This posture facilitates the escape of air from the nasal cavity, the ostiomeatal complex, and the paranasal sinuses (see FIG. 5). In this state, the outlet (33) of the adaptor (30) is inserted into the lower nostril and brought into close contact with the nostril (medication injection posture setting step). The medication stored in the medication storage container (20) is injected through the adaptor (30) into the nasal cavity. Because the volume of the nasal cavity differs from one person to another and varies with time or the state of disease, the injection is conducted just before the medication flows over into the oral cavity. For a healthy adult, the capacity of the nasal cavity amounts to 20 ml or more. For an adult affected by nasal obstruction, rhinitis, or sinus cold, the nasal cavity decreases in capacity and accommodates only 10 ml or less. When nasal obstruction becomes serious, the medication cannot be injected with a weak force. In this case, a very strong force should be slowly applied. In either case, the injection stops when a small amount of the medication starts to flow over into the mouth (medication injection step).

While the nasal cavity is full of the medication, the application and release of a pressure in the axial direction traversing the adaptor (30) is repeated using the medication storage container (20) to modulate the volume of the adaptor. The process of supplying the medication from the adaptor to the nasal cavity or vice versa is proceeded with the irregular change of the pressure (pressure application or release step). Thus, the medication delivered to the nasal cavity repeatedly undergoes counter flow and re-discharges to generate turbulence, vortex, pulsatile stream, or sloshing, whereby the medication can actively go to and withdraw from the intranasal cavity, the narrow turbinate region, the ostiomeatal complex of the paranasal sinuses, and the paranasal sinuses and thus can rinse the nasal cavity and inject the medication to the paranasal sinuses (pressure change-induced rinsing step in nasal cavity and paranasal sinuses).

In the pressure application and release step, the application of a pressure to the adaptor (30) and the release of the pressure is repeated at a frequency of 30-300 cycles per minutes and preferably at a frequency of 60-200 cycles per minutes for 1-5 minutes. This step is performed for each of the opposite nasal cavities. For serious infection or symptoms, such injection is preferably repeated within 30 minutes. However, this practice may vary in frequency and speed with symptoms. After the step of rinsing the nasal cavity and paranasal sinuses with pressure changes is conducted, the user may slightly blow his or her nose and temporarily plug the nostril with a sheet of tissue. Then, the user turns his or her face to the opposite side and lightly hits his or her head by hand so that the solution in the paranasal sinuses flows out of the nose.

The medication used is 0.6 to 5 w/v% brine or a 0.01-0.3% povidone-iodine solution and exhibits an excellent therapeutic effect on rhinitis, sinusitis, nasal discharge, nasal obstruction, sinus cold, or flu in the early stage. Having an anti-inflammatory effect as well as a disinfection effect, iodide-based medications can particularly alleviate inflammation and pain symptoms quickly in addition to killing infectious pathogens.

Therefore, when used to inject a medication, the device for injecting a medication into a nasal cavity and paranasal sinuses according to the present invention can prevent and treat diseases in the nasal cavity and paranasal sinuses, which are caused by various respiratory infections, such as common cold, flu, etc., and can effectively alleviate or remove the symptoms within a short period of time.

In particular, the device can be used for wiping out pathogens infecting the nasal cavity and paranasal sinuses, including: viruses, such as rhinovirus, coronavirus, influenza virus, adenovirus, human parainfluenza virus, respiratory syncytial virus, enterovirus other than rhinovirus, and metapneumovirus; and bacteria, such as Streptococcus pneumoniae, Haemophilus influenzae, Moraxella catarrhalis, Staphylococcus aureus and other streptococci strains which are a pathogenic bacteria causative of sinusitis, anaerobic bacteria, and Gram-negative bacteria.

### Example 1

For use in a device for injecting a medication into a nasal cavity and paranasal sinuses, the adaptor had a circular cone structure and was made of a soft elastic silicone material, with the dimensions: 0.3 mm for the connector diameter; 5 mm for connector length; about 3.5 ml for space volume; 8 mm for outlet inner diameter; 35 mm for total length; 22 mm for maximum diameter, and 2 mm for thickness.

The adaptor was mounted to a disposable syringe having a storage capacity of 20 ml and serving as the medication storage container.

### Comparative Example 1

A commercially available adaptor made of a hard plastic material lacking a compression and restoration potential was mounted to the same syringe as in Example 1.

### Test Example 1

### Treatment of infection into nasal cavity

The medication stored in the medication storage container was a 0.2 w/v % povidone-iodine solution and loaded in an amount of 15 ml to each syringe.

Selection was made of 40 subjects with sinus cold irrespective of sex and they were divided into two groups of 20 before the medication was injected thereto using the medication injection devices of Example 1 and Comparative Example 1.

For medication injection, a subject tilted his or her head by about 90 degrees to the front side and turned the face right or left by 90 degrees. While maintaining the posture, the subject inserted the outlet of the medication injection device into the lower nostril to achieve a close contact therebetween and then slowly pushed the piston of the syringe until the medication was about to flow over into the oral cavity. Afterwards, the subject applied a pressure toward the nasal cavity and released the compression at a frequency of 150 cycles for 2 minutes with the syringe held by his or her hand.

In Example 1, after medication injection, when the syringe was pressed against the nose, the residual medication in the adaptor entered the nasal cavity. When the syringe pressing was released, a part of the medication in the nasal cavity was withdrawn to the adaptor with the restoration of the compressed adaptor. As such, the compression and release of the adaptor was repeated at a high frequency for 2 minutes using the syringe. This repeated compression and release forced the medication to form irregular waves and streams, such as vortexes, turbulences, pulsatile streams, and sloshing, whereby the medication could enter secluded regions of the nasal cavity, such as the turbinate, and the paranasal sinuses.

For Comparative Example 1, the medication was incited to slightly flow only by the force applied to the nostril through the adaptor. In this regard, a significant amount of the medication leaked due to the loose contact between the adaptor and the nostril.

Medication injection was conducted for the opposite nasal cavity in the same manner.

For severe nasal infection and symptoms, the procedure was repeated within 30 minutes.

Irrespective of symptoms of the patients, Example 1 exhibited an effect of remarkably alleviating the infection in all of the patients, with almost no recurrence therein. This result indicates that the medication injection device and method of Example 1 is far superior in terms of injecting a medication into the nasal cavity, the ostiomeatal complex, and the paranasal sinuses and as such, can effectively rinse even deeply secluded regions in the nasal cavity.

In contrast, Comparative Example 1 exhibited a transitory effect of relieving the symptoms, but with near 70% of recurrence. This indicates that the medication could not sufficiently reach the nasal cavity and the paranasal sinuses and thus exhibited a poor effect.

Therefore, the device for injecting a medication to a nasal cavity and paranasal sinuses according to the present invention is designed to cause the medication to generate a turbulence, vortex, or sloshing in the nasal cavity through the compression and restoration thereof, whereby the injected medication can reach deeply secluded nasal paths and the paranasal sinuses to exhibit excellent pharmaceutical and rinsing effects.

In addition, the present invention can elicit an excellent symptom relieving effect even for common cold or flu.

### Test Example 2

### Test for nasal discharge and obstruction

As a liquid material stored in the medication storage container, 2.6% brine was used.

Selection was made of 40 subjects with nasal obstruction irrespective of sex and they were divided into two groups of 20 before the medication was injected thereto using the medication injection devices of Example 1 and Comparative Example 1.

The medication injection was carried out in the same manner as in Test Example 1.

Consistent to that of Test Example 1, the test result of Example 1 showed that nasal obstruction was almost removed from the test group to which the adaptor-mounted medication injection device of Example 1 was applied.

When the adaptor-mounted medication injection device of Comparative Example 1 was used, the nasal obstruction was removed temporarily, but appeared again within a short period of time. Therefore, far higher rinsing and medication injection effects were obtained in Example 1 than Comparative Example 1.

## Claims

1. A device for injecting a medication into the nasal cavity and the paranasal sinus through a nostril, the device comprising:
a) a medication storage container (20), having an inlet (21) at one side thereof, for containing the medication therein; and
b) an adaptor (30), comprising: an adaptor body (31), formed of one elastic material selected from silicone, rubber, and an elastic plastic material, for elastically moving to volumetrically reduce or restore the internal accommodating space (311) thereof depending on external pressures applied thereto; a connector (32) formed at the rear end of the adaptor body (31) and connected to the inlet (21) of the medication storage container (20); and an outlet (33), formed at the front end of the adaptor body, for forward discharging the medication provided from the medication storage container (20),
wherein while the nasal cavity is filled with the medication by injection through a nostril, pressure application and release are repeatedly carried out for the adaptor (30) to transfer the volume change of the internal accommodation space (311) of the adaptor (30) according to the pressure change to the medication in the nasal cavity so that the medication undergoes turbulence, vortex, pulsation, sloshing, or a pressure change, whereby the nasal cavity and the paranasal sinuses can be rinsed and administered the medication.

2. The device of claim 1, wherein the adaptor (30) has one of: a structure in which a front side protrudes to come into close contact with a nostril and a rear side of the adaptor has a wide heart form, a hemisphere form, a circular cone form, or a circular truncated cone form; a polygonal structure in which a front side is partially formed into a circular cone or a circular truncated cone; or a triangular or polygonal structure in which a front side is partially formed into a circular cone or a circular truncated cone, with the slant face of the circular cone or the circular truncated cone being concave or convex.

3. The device of claim 1 or 2, wherein the adaptor (30) is an integrated structure or an assembled structure that is detachably mounted horizontally or vertically.

4. The device of claim 1 or 2, wherein the adaptor body (31) of the adaptor (30) is formed of an elastic material and is 10-60 mm in length, 10-50 mm in maximum diameter, 1-50 ml in accommodation space volume, and 0.1-5 mm in thickness; the connector (32) is 1-5 mm in inner diameter and 0-10 mm in length; and the outlet (33) is 2-15 mm in diameter.

5. The device of claim 1 or 2, wherein the connector (32) of the adaptor is formed by perforating the adaptor body (31) or into an inwardly internalized structure or an outwardly protruding structure and is engaged with the inlet (21) of the medication storage container by screwing or press fitting.

6. The device of claim 1 or 2, wherein a plurality of reinforcement strips (312), each extending from the outlet (33) to the connector (32), is formed at regular distances of space on the inner surface of the adaptor (30) to reinforce a lengthwise elastic restoring force.

7. The device of claim 1 or 2, wherein the medication storage container (20) is a syringe or a compression container.

8. The device of claim 7, wherein the medication stored in the medication storage container (20) is an aqueous 0.6-5% NaCl solution or a solution containing povidone-iodine at a concentration of 0.01-0.3 w/v%.

9. A medication injection method using the medication injection device of claim 1, the method comprising:
a medication injection step in which a medication is injected into a nasal cavity through an adaptor by using a syringe containing the medication and serving as a medication storage container;
an adaptor compression and release step in which, while the nasal cavity is filled with the medication, pressures are repeatedly applied to and released from the syringe to discontinuously modulate the volume and pressure in the accommodation space (311) of the adaptor (30), thereby repeating the backward flow and reentry of the medication loaded to the nasal cavity; and
a rinsing step in which turbulence or sloshing occurs in the nasal cavity with the change of the medication stream in speed and pressure upon the backward flow and reentry, thereby rinsing the nasal cavity and paranasal sinuses.

10. The method of claim 9, the method further comprising a medication injection posture setting step in which the patient's head is tilted by 60-160 degrees to the lateral side, followed by injecting the medication through a lower nostril or in which the patient's head is tilted by 60-160 degrees to the front side and then the patient's face is turned by 60-160 degrees, followed by injecting the medication through a lower nostril, prior to the medication injections step.

11. The method of claim 9, wherein the application and release of pressures by the syringe in the adaptor compression and release step is carried out at a frequency of 30-300 cycles per minute.

12. The method of claim 9, wherein the medication injection is applied to the treatment or prevention of at least one of rhinitis, sinusitis, common cold, and influenza, thick nasal mucus, and nasal obstruction.
